# EUROPEAN PATENT APPLICATION

(11) **EP 1 500 401 A1**
(43) Date of publication of application: **26.01.2005**
(21) Application number: 03717687.2
(22) Date of filing: 22.04.2003
(51) Int. Cl.: A61K 45/00, A61K 31/27, A61K 31/695, A61P 9/00, A61P 9/10, A61P 9/14, A61P 43/00

(54) **DRUGS FOR TREATING VASCULAR DISEASES**

(30) Priority: 22.04.2002 JP 2002118729
(71) Applicant: Research Foundation Itsuu Laboratory, Tokyo 158-0094 (JP)
(72) Inventor: NAGAI, Ryozo, Bunkyo-ku, Tokyo 113-0033 (JP); SHINDO, Takayuki, Bunkyo-ku, Tokyo 113-0031 (JP); MANABE, Ichiro, Taito-ku, Tokyo 110-0008 (JP); SHUDO, Koichi, Suginami-ku, Tokyo 168-0073 (JP); KAGECHIKA, Hiroyuki, Nerima-ku, Tokyo 178-0062 (JP)
(74) Representative: Sternagel, Fleischer, Godemeyer & Partner
(86) International application number: PCT/JP2003/005084
(87) International publication number: WO 2003/089005

(57) **Abstract**

A medicament for prophylactic and/or therapeutic treatment of a vascular disease such as vascular restenosis and/or reocclusion after percutaneous transluminal coronary angioplasty using an intravascular stent, which comprises as an active ingredient a substance selected from the group consisting of retinoids and agents for controlling actions of retinoids such as 4-[5,6,7,8-tetrahydro-5,5,8,8-tetramethyl-2-naphthalenyl)carbamoyl]benzoic acid or a salt thereof, or 4-[[[3,5-bis-(trimethylsilyl)phenyl]carbonyl]amino]benzoic acid or a salt thereof, wherein said substance has substantially no antiproliferative action on vascular endothelial cells, but substantially has antiproliferative action on vascular smooth muscle cells.

## Description

### Technical Field

The present invention relates to a medicament for prophylactic and/or therapeutic treatment of a vascular disease and hypercardia.

### Background Art

Various medicaments such as antihypertensive agents, antihyperlipemia agents and antioxidants have been used for therapeutic treatments of vascular diseases such as arteriosclerosis and hypercardia. However, any of these medicaments achieve no better than symptomatic treatments, and currently no satisfactory therapeutic achievements have been obtained. Percutaneous transluminal coronary angioplasty (PTCA), which enables canalization and reconstruction of a stenosed blood vessel in angina pectoris or the like, has been developed, and treatments involving dilation with a balloon catheter and indwelling of a stent have been operated. However, problems arise in vascular damages caused upon the dilation with the balloon catheter and the indwelling of the stent, and restenosis and reocclusion caused by subsequent proliferation of smooth muscles. Some stents have been proposed which have a function of releasing a drug for therapeutic treatments of the conditions. However, they still have problems in low effectiveness and toxicity.

Sporn et al. reported a possibility of application of retinoids such as retinoic acid in therapeutic treatment of atherosclerosis (The American Journal of Medicine, 70, pp.1231-1236, 1981, in particular, page 1234, right column, lines 3-16). As for actions of retinoids on vascular smooth muscles, a review by Miano et al. (Circulation Research, pp.355-362, 2000) and a report by Neuville et al. (Arterioscler Tromb. Vasc. Biol., 1430-1436, 1999, in particular, Figure 6 and so forth) are published.

### Disclosure of the Invention

An object of the present invention is to provide a medicament for prophylactic and/or therapeutic treatment of a vascular disease or hypercardia, in particular, a medicament for prophylactic and/or therapeutic treatment of arteriosclerosis, a cerebrovascular disorder, a vascular disease due to intravascular physical injury and hypercardia. The inventors of the present invention conducted various researches to achieve the aforementioned object, and as a result, they found that retinoids such as 4-[5,6,7,8-tetrahydro-5,5,8,8-tetramethyl-2-naphthalenyl)carbamoyl]benzoic acid had substantially no antiproliferative action on vascular endothelial cells, whilst substantially had antiproliferative action on vascular smooth muscle cells, and that the substances had superior antiproliferative action on neointima of injured blood vessels, and markedly suppressed granulation. The present invention was achieved on the basis of the aforementioned findings.

The present invention thus provides a medicament for prophylactic and/or therapeutic treatment of a vascular disease, which comprises as an active ingredient a substance selected from the group consisting of retinoids and agents for controlling actions of retinoids, wherein said substance has substantially no antiproliferative action on vascular endothelial cells, but substantially has antiproliferative action on vascular smooth muscle cells.

According to preferred embodiments of the above invention, there are provided the aforementioned medicament, wherein the vascular disease is selected from the group consisting of arteriosclerosis, a cerebrovascular disorder, and a vascular disease due to intravascular physical injury; the aforementioned medicament, wherein the vascular disease due to the intravascular physical injury is vascular restenosis and/or reocclusion after percutaneous transluminal coronary angioplasty using an intravascular stent; the aforementioned medicament, which is contained in an intravascular stent or a balloon catheter in a form that enables a sustained release of said medicament; and the aforementioned medicament, wherein the substance as the active ingredient is 4-[5,6,7,8-tetrahydro-5,5,8,8-tetramethyl-2-naphthalenyl)carbamoyl]benzoic acid or a salt thereof, or 4-[[[3,5-bis(trimethylsilyl)phenyl]carbonyl]amino]benzoic acid or a salt thereof.

Further, from another aspect, the present invention provides a medicament for suppressing granulation due to intravascular physical injury, which comprises a substance selected from the group consisting of retinoids and agents for controlling actions of retinoids as an active ingredient. According to a preferred embodiment of this invention, there is provided the aforementioned medicament, wherein the substance substantially has antiproliferative action on neointima of an injured blood vessel. The present invention also provides a medicament for suppressing proliferation of neointima due to intravascular physical injury, which comprises a substance selected from the group consisting of retinoids and agents for controlling actions of retinoids as an active ingredient. The present invention further provides a medicament for prophylactic and/or therapeutic treatment of hypercardia, which comprises a substance selected from the group consisting of retinoids and agents for controlling actions of retinoids as an active ingredient, and a medicament for suppressing fibrosing of cardiac muscles in hypercardia, which comprises a substance selected from the group consisting of retinoids and agents for controlling actions of retinoids as an active ingredient. As preferred embodiments of these inventions, there are provided the aforementioned medicaments, wherein the substance as the active ingredient is 4-[5,6,7,8-tetrahydro-5,5,8,8-tetramethyl-2-naphthalenyl)carbamoyl]benzoic acid or a salt thereof, or 4-[[[3,5-bis(trimethylsilyl)phenyl]carbonyl]amino]benzoic acid or a salt thereof.

From further aspects, there are provided a method for prophylactic and/or therapeutic treatment of a vascular disease, which comprises the step of administering to a mammal including human a therapeutically effective amount of a substance selected from the group consisting of retinoids and agents for controlling actions of retinoids, wherein said substance has substantially no antiproliferative action on vascular endothelial cells, but substantially has antiproliferative action on vascular smooth muscle cells; a method for suppressing granulation due to intravascular physical injury, which comprises the step of administering an effective amount of a substance selected from the group consisting of retinoids and agents for controlling actions of retinoids to a mammal including human; and a method for suppressing proliferation of neointima due to intravascular physical injury, which comprises the step of administering an effective amount of a substance selected from the group consisting of retinoids and agents for controlling actions of retinoids to a mammal including human.

The present invention also provides a method for prophylactic and/or therapeutic treatment of hypercardia, which comprises the step of administering a prophylactically and/or therapeutically effective amount of a substance selected from the group consisting of retinoids and agents for controlling actions of retinoids to a mammal including human; and a method for suppressing fibrosing resulting from hypercardia, which comprises the step of administering an effective amount of a substance selected from the group consisting of retinoids and agents for controlling actions of retinoids to a mammal including human.

The present invention further provides an intravascular stent or an intravascular balloon catheter, which contains any of the aforementioned medicaments in a form that enables a sustained release of said medicament.

### Brief Explanation of Drawing

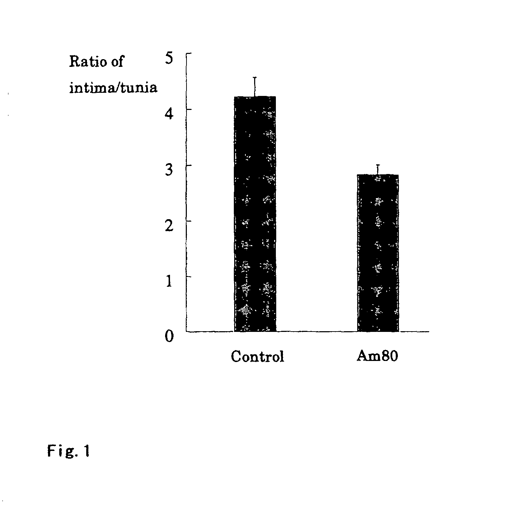

Fig. 1 shows restenosis suppressing effect of the medicament of the present invention (Am80) after indwelling of a stent.

### Best Mode for Carrying out the Invention

As the active ingredient of the medicaments of the present invention, retinoic acid and compounds having retinoic acid-like biological activities (these substances are generically called as "retinoids") can be used. As retinoic acid, for example, all-trans retinoic acid may be used. Further, various kinds of vitamin A derivatives synthesized so far such as the benzoic acid derivatives described in Japanese Patent Unexamined Publication (Kokai) Nos. (Sho) 61-22047/1986 and 61-76440/1986, and the compounds described in Journal of Medicinal Chemistry, 1988, Vol. 31, No. 11, p.2182 may be used as the active ingredient of the medicaments of the present invention.

More specifically, examples include 4-[5,6,7,8-tetrahydro-5,5,8,8-tetramethyl-2-naphthalenyl)carbamoyl]benzoic acid (hereinafter in the specification, this substance is referred to as "Am80"), 4-[[[3,5-bis(trimethylsilyl)phenyl]carbonyl]amino]benzoic acid (Tac101), 4-[5,6,7,8-tetrahydro-5,5,8,8-tetramethyl-2-naphthalenyl)carboxamido]benzoic acid (Am580), 6-[1-(5,6,7,8-tetrahydro -3,5,5,8,8-pentamethyl-2-naphthalenyl)cyclopropyl]pyridine-3-carboxylic acid (LG268), 5-[(5,6,7,8-(tetrahydro-5,5,8,8-tetramethyl-2-naphthalenyl)carboxamido]tropolone (Tp80) and the like. However, the compounds are not limited to these examples. Among them, Am80 and Tac101 are preferred, and particularly preferred compound is Am80.

As agents for controlling actions of retinoids, benzodiazepine derivatives such as 4-[5H-2,3-(2,5-dimethyl-2,5-hexano)-5-methyldibenzo[b,e][1,4]diazepin-11-yl]benzoic acid (HX600), and 4-[1,3-dihydro-7,8-(2,5-dimethyl-2,5-hexano)-2-oxo-2H-1,4-benzodiazepin-5-yl]benzoic acid (PCT/JP96/2709, International Patent Publication WO97/11061) are known. Although these compounds, per se, have no retinoid action or their retinoid actions are very weak, they have an action of remarkably enhancing actions of retinoids such as retinoic acid. A method for evaluation of the compounds enhancing retinoid actions is described in International Publication WO97/11061 (PCT/JP96/2709).

As agents for controlling actions of retinoid, compounds are also known to exist which have antagonistic action against retinoids and attenuate typical actions of the above retinoids (Eyrolles, L., et al., Journal of Medicinal Chemistry, 37(10), pp.1508-1517, 1994). This publication discloses that some compounds such as 4-(5H-7,8,9,10-tetra-hydro-5,7,7,10,10-pentamethylbenzo[e]naphtho[2,3-b][1,4]-diazepin-13-yl)benzoic acid (LE135) can act as antagonists of retinoids. Further, compounds including 4-(13H-10,11,12,13-tetrahydro-10,10,13,13,15-pentamethylnaphtho[2,3-b][1,2-e][1,4]diazepin-7-yl)benzoic acid are also known as agents for controlling actions of retinoids (specification of Japanese Patent Application No. (Hei) 7-255912/1995). Methods for evaluation of the compounds having antagonistic action against retinoids are described in the above publication by Eyrolles and the specification of Japanese Patent Application No. (Hei) 7-255912/1995.

Substances having the actions of retinoids as themselves can be preferably used as the active ingredient of the medicaments of the present invention. Further, HX600, LG268 and the like enhance actions of endogenous retinoids or pharmacologically administered retinoids, and therefore these substances can preferably be used as the active ingredient of the medicaments of the present invention. It is also possible to use a compound having antagonistic action against retinoids in combination to control the actions of retinoids in vivo. As the active ingredient of the medicaments of the present invention, a combination of two or more kinds of substances may be used. The substances exemplified above can be preferably used as the active ingredient of the medicaments of the present invention. However, the active ingredients of the medicaments of the present invention are not limited to these examples.

As the active ingredient of the medicaments of the present invention, physiologically acceptable acid addition salts or base addition salts may be used. Examples of the acid addition salts include mineral acid salts such as hydrochloride or hydrobromide, and organic acid salts such as p-toluenesulfonate, methanesulfonate, oxalate, or tartrate. Examples of the base addition salts include metal salts such as, for example, sodium salt, potassium salt, magnesium salt, and calcium salt, ammonium salts, or organic amine salts such as triethylamine salt and ethanolamine salt and the like. However, types of the salts are not limited to those exemplified above.

Further, when the active ingredient of the medicaments of the present invention has one or more asymmetric carbon atoms depending on types of substituents, any stereoisomers on the basis of the asymmetric carbon atoms such as optical isomers and diastereomers in pure forms, any mixtures of stereoisomers, racemates and the like may be used as the active ingredient of the medicaments of the present invention. Furthermore, hydrates or solvates of compounds in free forms or salt forms may also be used as the active ingredient of the medicaments of the present invention.

The medicament of the present invention can be used for prophylactic and/or therapeutic treatment of a vascular disease. Types of the vascular diseases are not particularly limited. Examples include, for example, arteriosclerosis, a cerebrovascular disorder such as cerebrovascular sclerosis, a vascular disease due to intravascular physical injury and the like. When the medicament of the present invention is used for the aforementioned purpose, the retinoids and agents for controlling actions of retinoids as the active ingredient are preferably the substances which have substantially no antiproliferative action on vascular endothelial cells, but substantially have antiproliferative action on vascular smooth muscle cells. Further, it is also preferred that they are substances having differentiation-modifying action. A typical example of particularly preferred retinoid having the aforementioned property is Am80. One or ordinary skill in the art can pharmacologically examine whether or not a certain substance "has substantially no antiproliferative action on vascular endothelial cells, but substantially has antiproliferative action on vascular smooth muscle cells" by, for example, the method described in Example 1.

Preferred objects of application of the medicaments of the present invention include arteriosclerosis due to various kinds of causes, as well as vascular restenosis, and reocclusion after percutaneous transluminal coronary angioplasty using an intravascular stent or an intravascular balloon catheter. When the medicament of the present invention is used for the purpose mentioned above, the substance as the active ingredient preferably has an action of suppressing granulation due to intravascular physical injury and substantially has an antiproliferative action on neointima of an injured blood vessel. A typical example of particularly preferred retinoid having the aforementioned property is Am80.

Furthermore, the medicament of the present invention can be used for prophylactic and/or therapeutic treatment of hypercardia. The medicament of the present invention can suppress proliferation of fibrous tissues with inflammation, such as fibrosing of interstitium and fibrosing of peripheries of coronary artery resulting from hypercardia. The terms herein used as for the objective diseases (for example, vascular disease, hypercardia, fibrosing and the like) should not be construed in any limitative way, and they should be construed in their broadest senses.

As the medicaments of the present invention, one or more kinds of substances per se, which are selected from the group consisting of retinoids, agents for controlling actions of retinoids and physiologically acceptable salts thereof as well as hydrates thereof and solvates thereof, may be administered. They can be preferably administered in the form of a pharmaceutical composition comprising the aforementioned one or more kinds of substances and one or more kinds of pharmaceutical additives. The aforementioned pharmaceutical composition may also be further added with one or more kinds of active ingredients of other medicaments and used as a pharmaceutical composition in the form of so-called combination preparation. Types of the pharmaceutical additives are not particularly limited. Examples of the pharmaceutical additives include excipients, disintegrators or disintegrating aids, binders, lubricants, coating agents, colorants, diluents, base materials, dissolving agents or solubilizers, isotonic agents, pH modifiers, stabilizers, propellants, adhesives and the like.

The medicaments of the present invention can be administered orally or parenterally. Examples of the pharmaceutical compositions suitable for oral administration include, for example, tablets, capsules, powders, subtilized granules, granules, liquids, syrups and the like. Examples of the pharmaceutical compositions suitable for parenteral administration include, for example, injections, drops, suppositories, inhalants, transdermally absorbable preparations, transmucosally absorbable preparations and the like. The medicament of the present invention can be used with angiotensin II receptor antagonists, calcium antagonists, ACE inhibitors, antihypercholesteremia agents, or other circulatory drugs. Doses of the medicaments of the present invention can appropriately be chosen depending on the type of the disease to be treated, the age and body weight of a patient, severity of the disease, the type of the active ingredient and the like. For example, when Am80 is orally administered as the active ingredient, the dose is about 0.1 to 30 mg per day for adults.

The intravascular stent and the intravascular balloon catheter provided by the present invention can release the aforementioned substances as the active ingredient of the medicament of the present invention into blood. As a result, they can suppress granulation due to intravascular physical injury caused by indwelling of a stent or a surgical operation using an intravascular balloon catheter, and can exhibit proliferation-promoting action on neointima of injured blood vessels.

Base materials for preparing the stent are not particularly limited. Stainless steel (SUS316, SUS304), Nitinol (Ni-Ti alloy), metallic materials such as tantalum and polymer materials can be generally used, and biodegradable polymer materials can also be used. As for the polymer materials, types of the materials are not particularly limited so far that they have blood compatibility and are not dissolvable in blood. The method for producing the stent of the present invention is not particularly limited. For example, when the stent base material consists of a metal, a polymer coating layer containing the medicament of the present invention can be provided on the surface of the stent base material, or when the base material consists of a polymer material, the medicament of the present invention may be introduced into the polymer material upon molding thereof, or a polymer coating layer containing the medicament of the present invention can be provided on the surface of the stent base material.

Types of the polymer materials to form the coating layer are not particularly limited so far that the materials have blood compatibility and are not dissolvable in blood. For example, polyester type elastomers, polyamide type elastomers, polyurethane type elastomers, (meth)acrylate ester type polymers, polyvinyl acetates, poly(ethylene-vinyl alcohol) copolymers and the like can be used. A polymer material having compliance respondable to expansion of the stent is more desirable.

Concentrations of the aforementioned active ingredient and the aforementioned polymer in a solution for coating can be suitably chosen depending on conditions including, for example, an amount to be eluted (elution rate) of the aforementioned substances from a surface of the coated layer and a shape of the stent. The stent is desirably designed so as to have a sustained release property in such a degree that the effectiveness of the medicament of the present invention is maintained for at least a given period of time. It is generally desirable to design the stent so that a local concentration of the active ingredient can be 10 µM to 1 nM.

Methods for producing a stent and means for sustained release are well known and conventionally used by artisans in the fields of stents, artificial organs and the like. For example, as for drug-releasing stents for prevention of restenosis, a review by Kozuma is published (Kozuma, K., Coronary Intervention, Vol. 1, pp.58-62), and specific drug-releasing stents are described in Catheterization and Cardiovascular Interventions, Vol. 55, pp.409-417, 2002; New England Journal of Medicine, Vol. 346, 1770-1771 and 1773-1780, 2002; W002/064065 and the like. The entire disclosures of these publications and the publications cited therein are incorporated by reference in the disclosures of the specification. By referring to these publications, those skilled in the art can readily produce the intravascular stent of the present invention. The intravascular balloon catheter of the present invention can also be readily produced by suitably applying these known techniques.

### Examples

The present invention will be more specifically explained with reference to the following examples. However, the scope of the present invention is not limited to these examples.

### Example 1: Effect on proliferation of endothelial cells and smooth muscle cells

Rat aorta smooth muscle cells and human umbilical artery endothelial cells were cultured in the presence of 10% fetal bovine serum, and added with Am80 at various concentrations. After 24 hours, BrdU was added to the culture medium, and BrdU uptake ability was measured for 4 hours. The ratios of uptake of BrdU at the various concentrations relative to the uptake obtained without addition of Am80 are summarized in the table. The BrdU uptake ability represents DNA synthesis ability in proportion to the proliferation.

**Table 1**

| | Am80 Concentration | | | | |
|---|---|---|---|---|---|
| Cell | 0 µM | 0.3 µM | 1 µM | 3 µM | 10 µM |
| Endothelial cell | 100.0 | 95.1 | 109.4 | 104.0 | 102.1 |
| Smooth muscle cell | 100.0 | 95.5 | 78.5 | 74.4 | 68.8 |

### Example 2: Pachymenia of vascular adventitia and neointima in cuff-injured femoral artery model

Polyethylene tube cuffs were indwelled in femoral arteries of wild-type mice (129SVxC57BL6) to injure the arteries. Am80 was orally administered at a dose of 5 mg/kg/day, and after 5 weeks, appearance and cross sections of the injured sites were evaluated. Areas of neointima of the femoral artery covered with the tube cuff and granulation tissue around the cuff were measured. As a result, remarkable formation of granulation tissues was observed in the mice not administered with the medicament, in such a degree that ligatures used for ligation of the polyethylene cuffs was not observable, whereas the granulation was markedly suppressed in the mice of the medicament-treated group. The results of numerically expressed evaluation are shown in Table 2. Neointima and formation of granulation tissues were significantly decreased in the Am80-administered mice compared with the control mice (no administration of the medicament).

**Table 2**

| | Control mice | Am80-administered mice |
|---|---|---|
| Neointima | 0.0117 mm² | 0.0019 mm² |
| Granulation tissue | 1.08 mm² | 0.44 mm² |

It is known that various kinds of growth factors are induced in blood vessel walls against affections in the cardiovascular system remodeling. By using mice (KLF5/BTEB2 +/- mice) with hetero-knockout of Zn finger type transcription factor KLF5/BTEB2, which is strongly expressed in neointima of injured blood vessels among such growth factors, studies were made to know whether or not the effect of proliferation of neointima was induced by the retinoid action. In these knockout mice, areas of hyperplasia sites were extremely decreased, and thus granulation was suppressed. When a retinoid antagonist LE135 (a substance acting on retinoids in a suppressive manner) was administered to these model mice, strong granulation was observed. From these results, it is readily understood that the medicament of the present invention suppressed proliferation of neointima, and suppressed granulation at the same time.

### Example 3: Effect of suppressing restenosis after indwelling of stent

Internal diameter of a rabbit common iliac artery was measured by intravascular ultrasound imaging (IVUS), and then a balloon was expanded to a size of 1 to 1.1 times of the internal diameter and a stent was indwelled. After the indwelling, the internal diameter was measured again by IVUS to confirm that the internal diameter changed 1 to 1.1 times and the stent was precisely indwelled. For each of the control group and Am80-administrated group, 6 rabbits were used. The rabbits were orally administered with Am80 at a dose of 1 mg/kg every day, and 4 weeks after the indwelling of the stent, the arteries at the stent-indwelled sites were collected and fixed to examine tissue images. Serial sections were prepared for the proximal, intermediary, and distal positions within the stent, and areas of tunica media and intima were measured. The results are shown in Fig. 1. The ratio of intima/tunica media was significantly decreased in the Am80-administrated group (p<0.05).

### Example 4: Suppression of hypercardia

Mice were administered with angiotensin II at a dose of 3.2 mg/kg/day for two weeks by using an osmotic pressure pump to prepare hypercardia models. After 2 weeks, hypercardia, stromal fibrosing, and fibrosing around coronary artery were observed. In mice administered with angiotensin II and Am80 (5 mg/kg/day) in the same manner for two weeks, onsets of these pathological conditions were markedly suppressed.

**Table 3**

| | Control mice | Am80-administered mice |
|---|---|---|
| Heart weight/body weight | 5.65 ± 0.195 mg/g | 4.78 ± 0.170 mg/g |

### Industrial Applicability

The medicaments of the present invention are useful for prophylactic and/or therapeutic treatment of vascular diseases such as arteriosclerosis, cerebrovascular disorders, and vascular diseases due to intravascular physical injury and hypercardia.

## Claims

1. A medicament for prophylactic and/or therapeutic treatment of a vascular disease, which comprises as an active ingredient a substance selected from the group consisting of a retinoid and an agent for controlling an action of a retinoid, wherein said substance has substantially no antiproliferative action on vascular endothelial cells, but substantially has antiproliferative action on vascular smooth muscle cells.

2. The medicament according to claim 1, wherein the vascular disease is selected from the group consisting of arteriosclerosis, a cerebrovascular disorder, and a vascular disease due to intravascular physical injury.

3. The medicament according to claim 2, wherein the vascular disease due to intravascular physical injury is vascular restenosis and/or reocclusion after percutaneous transluminal coronary angioplasty using an intravascular stent.

4. The medicament according to claim 3, which is contained in an intravascular stent or a balloon catheter in a form that enables a sustained release of said medicament.

5. The medicament according to any one of claims 1 to 4, wherein the substance as the active ingredient is 4-[5,6,7,8-tetrahydro-5,5,8,8-tetramethyl-2-naphthalenyl)carbamoyl]benzoic acid or a salt thereof, or 4-[[[3,5-bis(trimethylsilyl)phenyl]carbonyl]amino]benzoic acid or a salt thereof.

6. A medicament for suppressing granulation due to intravascular physical injury, which comprises a substance selected from the group consisting of a retinoid and an agent for controlling an action of a retinoid as an active ingredient.

7. The medicament according to claim 6, wherein the substance has substantial antiproliferative action on neointima of an injured blood vessel.

8. A medicament for suppressing proliferation of neointima due to intravascular physical injury, which comprises a substance selected from the group consisting of a retinoid and an agent for controlling an action of a retinoid as an active ingredient.

9. A medicament for prophylactic and/or therapeutic treatment of hypercardia, which comprises a substance selected from the group consisting of a retinoid and an agent for controlling an action of a retinoid as an active ingredient.

10. A medicament for suppressing fibrosis in hypercardia, which comprises a substance selected from the group consisting of a retinoid and an agent for controlling an action of a retinoid as an active ingredient.

11. The medicament according to any one of claims 6 to 10, wherein the substance as the active ingredient is 4-[5,6,7,8-tetrahydro-5,5,8,8-tetramethyl-2-naphthalenyl)carbamoyl]benzoic acid or a salt thereof, or 4-[[[3,5-bis(trimethylsilyl)phenyl]carbonyl]amino]benzoic acid or a salt thereof.

12. An intravascular stent or an intravascular balloon catheter, which contains the medicament according to any one of claims 6 to 8 in a form that enables a sustained release of said medicament.
